# EUROPÄISCHE PATENTANMELDUNG

(11) **EP 1 536 249 A1**
(43) Veröffentlichungstag der Anmeldung: **01.06.2005**
(21) Anmeldenummer: 03405847.9
(22) Anmeldetag: 26.11.2003
(51) Int. Cl.: G01T 1/161, A61B 6/00

(54) **Vorrichtung zum Erzeugen von Echtzeit-Röngtgenbildern und Röntgengerät**

(71) Anmelder: Swissray Medical AG, 6280 Hochdorf (CH)
(72) Erfinder: Laupper, Rudolf G., 6285 Hitzkirch (CH)
(74) Vertreter: Wenger, René

(57) **Zusammenfassung**

Vorrichtung zum Erzeugen von Echtzeit-Röntgenbildern mit einem sich in einer Bildaufnahmeebene erstreckenden, flächigen Gehäuse (1), in welchem ein digitaler Röntgenbildsensor (2) angeordnet ist. Zudem umfasst die Vorrichtung eine Halterung (3) für die Montage des Gehäuses (1) an ein Röntgengerät mit einer Strahlenquelle (13). Diese Halterung (3) weist Gelenkmittel zum Verstellen des Gehäuses (1) zwischen einer Ruhestellung und einer Arbeitsstellung auf, wobei das Gehäuse (1) in der Arbeitsstellung im Strahlenfeld der Strahlenquelle positioniert ist.

## Beschreibung

Die Erfindung betrifft eine Vorrichtung zum Erzeugen von Echtzeit-Röntgenbildern sowie ein Röntgengerät mit einer Vorrichtung zum Erzeugen von Echtzeit-Röntgenbildern gemäss den Patentansprüchen 1 und 9.

In der medizinischen Radiologie kommen immer häufiger digitale Röntgengeräte zum Einsatz. Ein solches Gerät ist z.B. in EP 1 063 537 beschrieben. Die Digitalisierung der Daten kann entweder durch ein separates Abtasten oder mittels einer CCD Kamera online erfolgen. Die Datenaufnahme verläuft schnell, keine lange Entwicklungszeit des Röntgenbildes ist notwendig. Durch die Möglichkeit der digitalen Datenaufbewahrung lässt sich zudem die Aktenverwaltung erheblich vereinfachen.

Für einige Anwendungen ist es von Vorteil, statt Einzelaufnahmen eine Echtzeit-Röntgenüberwachung am Patienten vorzunehmen. Mit Röntgenbildsensoren ist dies nun in digitaler Weise möglich.

Für Ärzte, die sowohl Einzelaufnahmen als auch Echtzeitüberwachungen einsetzen möchten, wäre ein Röntgengerät von Vorteil, das in einfacher Weise zwischen den beiden Betriebsarten umstellbar ist. Dies ist bisher nicht realisiert worden.

Es ist daher eine Aufgabe der vorliegenden Erfindung, eine Vorrichtung für ein Röntgengerät zu schaffen, mit der in einfacher Weise zwischen einer Einzelaufnahme-Einstellung und einer Echtzeitüberwachung-Einstellung gewechselt werden kann. Ausserdem sollen mit dieser Vorrichtung auch bestehende Röntgengeräte nachgerüstet werden können. Die Vorrichtung soll zudem einen hohen Grad von Betriebssicherheit gewährleisten.

Diese Aufgabe wird mit der Vorrichtung zum Erzeugen von Echtzeit-Röntgenbildern und mit dem Röntgengerät gemäss den Patentansprüchen 1 und 9 gelöst.

Die Vorrichtung besteht aus einem Gehäuse, in dem ein digitaler Röntgenbildsensor angeordnet ist. Das Gehäuse ist grundsätzlich flächig und vorzugsweise rechteckig ausgebildet und erstreckt sich in einer Bildaufnahmeebene. Zudem umfasst die Vorrichtung eine Halterung für die Montage des Gehäuses an ein Röntgengerät mit einer Strahlenquelle. Diese Halterung weist Gelenkmittel auf, mit welchen das Gehäuse zwischen einer Ruhestellung und einer Arbeitsstellung verstellt wird. In der Arbeitsstellung befindet sich das Gehäuse, insbesondere die Bildaufnahmeebene, im Strahlenfeld der Strahlenquelle.

In einer bevorzugten Ausführungsform umfassen die Gelenkmittel ein erstes und ein zweites Gelenk. Am ersten Gelenk ist das Gehäuse um eine Achse schwenkbar, die vorzugsweise senkrecht zur Bildaufnahmeebene liegt, wenn sich das Gehäuse in der Ruhestellung befindet. Die Achse verläuft dabei ausserhalb des Gehäuses. Am zweiten Gelenk kann das Gehäuse um eine Achse senkrecht zur ersten Achse geschwenkt werden. Die Gelenke können zum Beispiel als Wälzlager, als Scharniere oder als Kugelgelenke in verschiedenen Kombinationen ausgebildet sein.

Die zweite Schwenkachse verläuft vorzugsweise parallel zur Bildaufnahmeebene und/oder zu einer Seite des Gehäuses.

Der Abstand der zweiten Schwenkachse zum Gehäuse beträgt vorzugsweise weniger als 300 mm. In einer weiteren bevorzugten Ausführungsform ist das Gehäuse über eine Linearführung mit der Halterung verbunden, so dass es relativ zur zweiten Schwenkachse zwischen einer ausgezogenen und einer eingefahrenen Position verstellbar ist. In der Betriebsstellung kann somit der Abstand des Gehäuses zur zweiten Schwenkachse vergrössert oder flexibel eingestellt werden. In der Ruhestellung oder während der Schwenkbewegung wird das Gehäuse vorzugsweise eingefahren. Insbesondere wird während der Schwenkbewegung damit die Kraft reduziert, die zum Schwenken des Gehäuses notwendig ist, die Ausrichtung in der Arbeitsstellung hingegen vereinfacht. Die Ausfahrdistanz beträgt vorzugsweise ca. 80 bis 110 mm.

Die Linearführung kann so ausgebildet sein, dass das Gehäuse nur zwischen zwei Positionen (eingefahren bzw. ausgefahren) verstellbar ist. Es ist aber auch möglich, eine Führung zu realisieren, bei der das Gehäuse Zwischenpositionen einnehmen oder sogar kontinuierlich verstellt werden kann. Die einfachste Ausführungsform für eine Linearführung besteht aus einer oder mehreren Schienen oder Stangen, entlang welchen das Gehäuse bewegt wird. Aber z.B. Teleskoparme oder Gewindespindeln können sich ebenfalls zur Realisierung der Linearführung eignen.

Vorzugsweise lässt sich das Gehäuse um beide Achsen um mindestens 90° drehen.

In einer besonders bevorzugten Ausführungsform ist das Gehäuse während der Ruhestellung an der Halterung feststellbar. Damit reduziert sich die Gefahr, dass das Gehäuse oder der Röntgenbildsensor beschädigt werden, solange sie nicht gebraucht werden. Zum Feststellen des Gehäuses kann z.B. eine Klammer an der Halterung befestigt sein, in welche das Gehäuse durch eine Schwenkbewegung entlang der zweiten Schwenkachse gedrückt wird. Ebenso ist es denkbar, dass das Gehäuse mittels einer Schraube oder eines Riegels fixiert wird.

Weiter ist es vorteilhaft, wenn die Halterung auch Mittel zum Fixieren des Gehäuses in der Arbeitsstellung aufweist. Auch dies können Klammern, Riegel oder Schrauben sein. Für die Fixierung in einer oder beiden Stellungen ist aber auch der Einsatz von Magneten, insbesondere Permanentmagneten denkbar.

Alternativ zu einer manuellen Betätigung können die Schwenkbewegungen und/oder das Ein- und Ausfahren des Gehäuses motorisiert durchgeführt und extern gesteuert werden.

Ein Röntgengerät umfasst eine Strahlenquelle, die Röntgenstrahlen aussendet, und ein Gestell, an dem ein Bucky zum Erzeugen digitaler Röntgenbilder angebracht ist. Das Bucky ist derart angeordnet, dass die Empfangsfläche des Bucky auf den Strahlengang der Strahlenquelle ausrichtbar ist. Zudem ist am Gestell eine Vorrichtung zum Erzeugen von Echtzeit-Röntgenbildern wie oben beschrieben angebracht. In der Arbeitsstellung ist das Gehäuse der Vorrichtung so im Strahlenfeld der Strahlenquelle angeordnet, dass die Bildaufnahmeebene etwa planparallel zur Empfangsfläche des Bucky verläuft.

Vorzugsweise ist das Bucky um eine etwa horizontale Achse drehbar am Gestell gelagert. Die erste Gelenkachse für das Gehäuse verläuft parallel zur Drehachse des Bucky oder fällt mit dieser zusammen. Dabei ist es vorteilhaft, wenn das Bucky über einen Motor, z.B. einen Elektromotor gedreht wird.

Besonders vorteilhaft ist die Vorrichtung zum Erzeugen von Echtzeit-Röntgenbildern so am Gestell angebracht, dass in der Arbeitsstellung der Vorrichtung das Bucky und das Gehäuse nur gemeinsam schwenk- bzw. drehbar sind. Ebenso wäre es möglich, dass auch während der Ruhestellung das Gehäuse mitbewegt wird, wenn das Bucky gedreht wird.

In einer weiteren Ausführungsform verfügt die Vorrichtung zum Erzeugen von Echtzeit-Röntgenbildern über Arretierungsmittel, mit denen sie zumindest in der Ruhestellung und/oder in der Arbeitsstellung arretierbar ist. Diese Arretierung wird über eine Steuerungseinrichtung betätigt. Wenn das Bucky über einen Motor gedreht wird, steht diese Steuerungseinrichung derart mit dem Motor oder gegebenenfalls mit einer Bremse in Wirkverbindung, dass die Arretierung der Vorrichtung bei Motorbetätigung sofort gelöst wird. Damit wird verhindert, dass das Gehäuse während der Drehung des Bucky arretiert bleibt und durch dessen Krafteinwirkung beschädigt wird.

Ein Ausführungsbeispiel der Erfindung ist in den Zeichnungen dargestellt und wird nachstehend genauer beschrieben. Es zeigen:
- Fig. 1:: eine perspektivische Darstellung der Vorrichtung in Ruhestellung,
- Fig. 2a:: eine Darstellung der Vorrichtung in Arbeitsstellung mit eingeschobenem Gehäuse,
- Fig. 2b:: die Darstellung gemäss Fig. 2a mit ausgezogenem Gehäuse,
- Fig. 3:: Aufsicht auf die Vorrichtung in Arbeitsstellung,
- Fig. 4:: Schnitt durch die Vorrichtung gemäss Fig. 3 entlang der Linie A-A,
- Fig. 5:: Schnitt durch die Vorrichtung gemäss Fig. 3 entlang der Linie B-B,
- Fig. 6:: Schnitt durch die Vorrichtung gemäss Fig. 3 entlang der Linie D-D,
- Fig. 7:: eine perspektivische Darstellung des Röntgengeräts mit Gestell und Bucky und mit einer erfindungsgemässen Vorrichtung in Ruhestellung,
- Fig. 8:: Die Vorrichtung gemäss Fig. 7 mit der erfindungsgemässen Vorrichtung in Arbeitsstellung,
- Fig. 9:: eine perspektivische Darstellung eines Röntgengeräts wie in Fig. 7 mit einem C-Arm und einer Strahlenquelle,
- Fig. 10:: das Röntgengerät gemäss Fig. 9 mit der erfindungsgemässen Vorrichtung in Arbeitsstellung,
- Fig. 11:: eine schematische Darstellung der Arretierungsmittel der Vorrichtung in Wirkverbindung mit dem das Bucky antreibenden Motor, und
- Fig. 12:: eine perspektivische Detailansicht des Gehäuses.

Der Aufbau der Vorrichtung wird zunächst anhand der Figuren 3 bis 6 erläutert. Der eigentliche Röntgenbildsensor 2 erstreckt sich in einer Bildaufnahmeebene B und ist in einem etwa rechtekkigen Gehäuse 1 untergebracht. Dieses Gehäuse ist vorzugsweise aus zwei Schalen zusammengesetzt, die aus Metall und/oder aus Kunststoff bestehen können. Die beiden parallelen Längsseiten des Gehäuses sind etwas angeschrägt. An seinem freien Ende ist das Gehäuse mit einer Griffmulde 20 versehen, um dessen Handhabung zu erleichtern. Schalt- oder Kontrollelemente 19 können im Bereich der Griffmulde angeordnet sein.

Das Gehäuse ist über nachstehend noch genauer beschriebene Gelenkmittel mit einer Halterung 3 verbunden. Diese besteht im wesentlichen aus einer Hohlwelle 15, welche fest mit einem Bucky 10 (Fig. 7 - 10) verbindbar ist. Um diese Hohlwelle ist mit Hilfe von speichenartigen Verstrebungen ein Führungsring 14 angeordnet. Auf diesem sitzt ein Gelenksattel 21, der mittels Wälzlagern 16 um den Mantel des Führungsrings gedreht werden kann. Zu diesem Zweck sind die dem Gelenksattel 21 zugeordneten Wälzlager sowohl gegen den Aussenmantel als auch gegen den Innenmantel des Führungsrings gespannt, so dass eine spielfreie Bewegung möglich ist.

Der Gelenksattel 21 und der Führungsring 14 bilden ein erstes Drehgelenk 6, mit dessen Hilfe das Gehäuse 1 um eine erste Schwenkachse S1 geschwenkt werden kann.

Im oberen Bereich des Gelenksattels ist mit Hilfe eines Scharnierbolzens 23 ein Scharnierteil 22 angelenkt. Dadurch wird ein zweites Gelenk 5 gebildet, um dessen Schwenkachse S2 das Gehäuse 1 schwenkbar ist. Der Schwenkwinkel α1 zwischen der Ruhestellung und der Arbeitssttellung A beträgt 90°. Ein zusätzlicher Schwenkwinkel α2 von ca. 10° dient dazu, dass das Gehäuse 1 in eine Halteklammer 12 (Fig. 7, 8) eingerastet werden kann.

Zwischen dem Gehäuse 1 und dem Scharnierteil 22 ist eine Linearführung L wirksam, mit deren Hilfe die Distanz zwischen dem Gehäuse 1 und der zweiten Schwenkachse S2 verändert werden kann. Diese Linearführung besteht beim Ausführungsbeispiel aus ingesamt drei Führungsstangen 4, von denen in Fig. 6 eine im Schnitt dargestellt ist. Von den drei Führungsstangen liegen nur die beiden äusseren auf der gleichen Ebene, während die mittlere auf einer etwas tieferen Ebene angeordnet ist. Dadurch wird eine gewisse Stützwirkung erzielt. Die Führungsstangen laufen beispielsweise in Kugelführungen oder in Gleitlagern. Wie insbesondere aus Fig. 12 ersichtlich ist, ist die gesamte Linearführung L von ineinander geschobenen Gehäuseteilen umgeben und damit von aussen nicht sichtbar.

Zur Fixierung des Gehäuses 1 in der Arbeitsposition A ist auf der Hohlwelle 3 in der Nähe der zweiten Schwenkachse S2 ein Permanentmagnet 7 angeordnet. Auf der Unterseite des Gehäuses 1 ist eine Gegenpolplatte 8 vorgesehen, so dass das Gehäuse 1 in der Arbeitsstellung mit einer bestimmten Magnetkraft festgehalten wird.

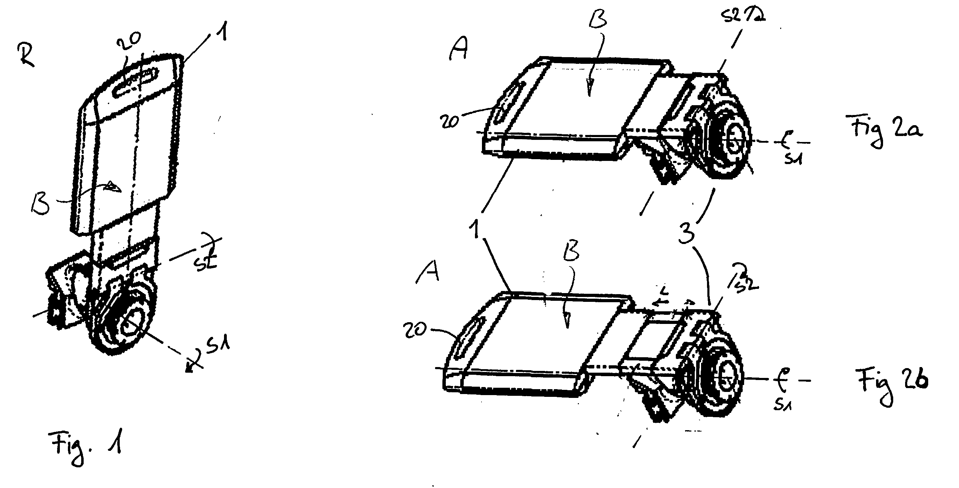

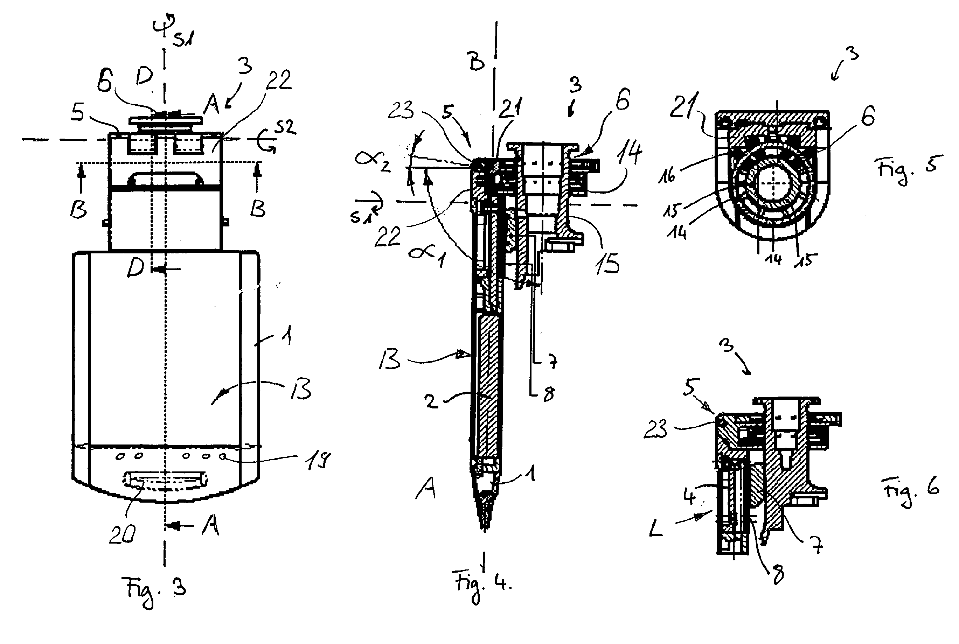

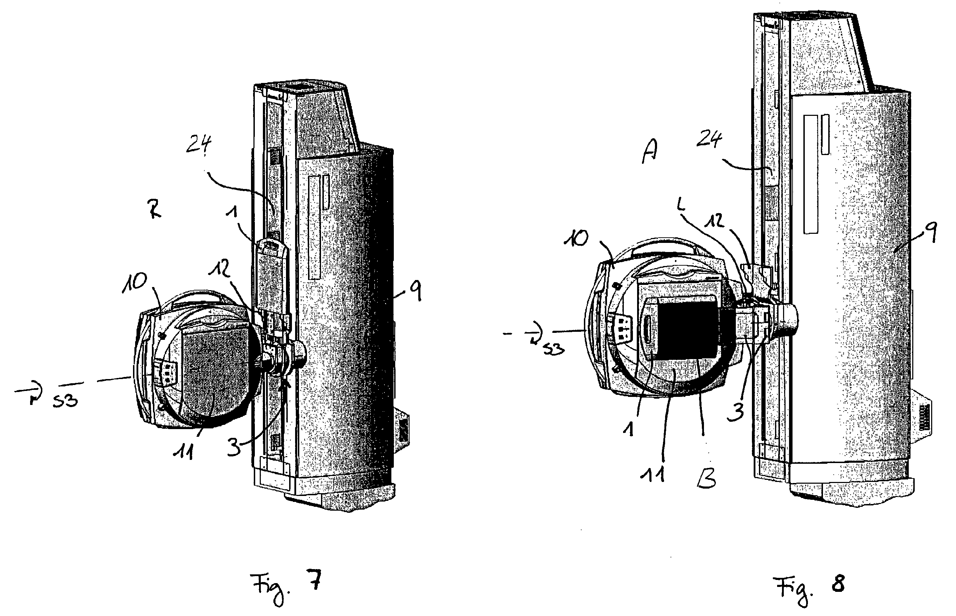

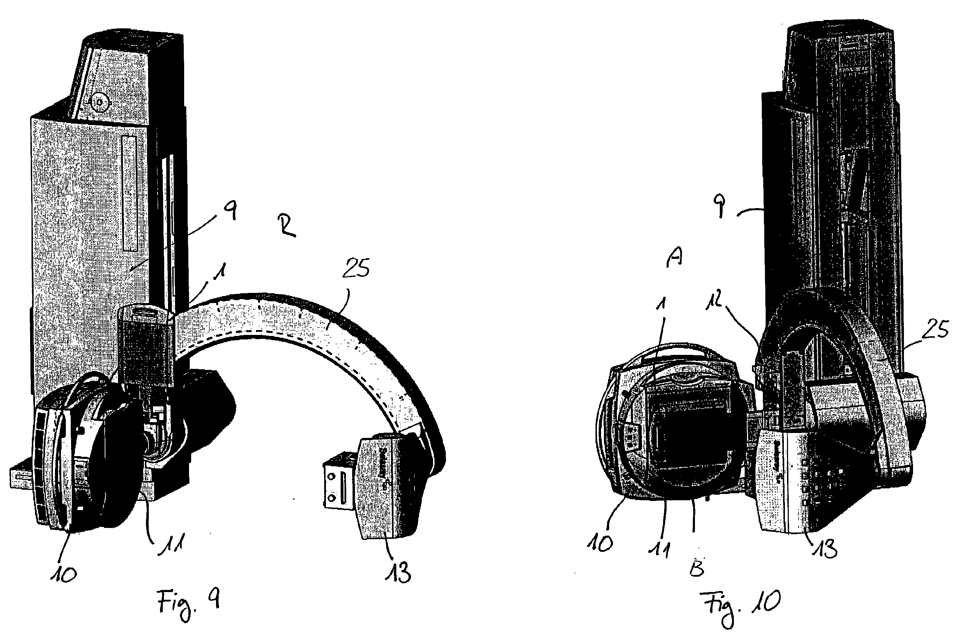

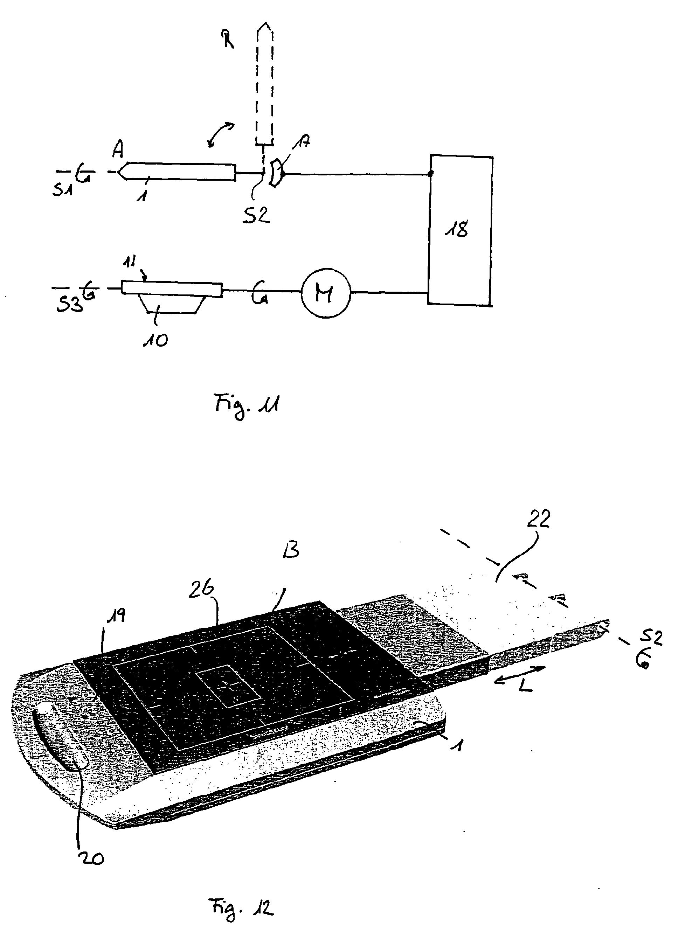

Fig. 1 zeigt das Gehäuse 1 in Ruhestellung R, also mit einer Bildaufnahmeebene B, die etwa im rechten Winkel zur ersten Schwenkachse S1 verläuft. Die Fig. 2a und 2b zeigen das Gehäuse 1 in Arbeitsstellung A, also mit einer Bildaufnahmeebene B, die etwa parallel zur ersten Schwenkachse S1 verläuft. In Fig. 2A befindet sich das Gehäuse 1 in der eingeschobenen und in der Fig. 2B in der ausgezogenen Position. Der Verschiebeweg beträgt ca. 90 mm.

Die Fig. 7 und 8 zeigen ein Röntgengerät ohne fest zugeordnete Strahlenquelle. Diese könnte beispielsweise an einem separaten Arm an der Decke angeordnet sein. Das Röntgengerät besteht im wesentlichen aus einem Gestell 9, an welchem entlang einer Vertikalführung 24 ein Bucky 10 höhenverstellbar angeordnet ist. Das Bucky mit seiner Bildempfangsfläche 11 ist ausserdem um eine Schwenkachse S3 schwenkbar. Diese Schwenkbewegung erfolgt um die Hohlwelle 15 gemäss den Fig. 4 und 5. Die Halterung 3 der Vorrichtung zum Erzeugen von Echtzeit-Röntgenbildern ist ersichtlicherweise zwischen dem drehbaren Bucky 10 und dem Gestell 9 angeordnet. Gemäss Fig. 7 ist das Gehäuse 1 in der Ruhestellung R angeordnet. Dabei ist es in einer Halteklammer 12 eingerastet, welche der Halterung 3 zugeordnet ist. Am Röntgengerät wird jetzt ausschliesslich mit dem Bucky 10 gearbeitet.

Für das Erzeugen von Echtzeit-Röntgenbildern wird das Gehäuse 1 gemäss Fig. 8 in die Arbeitsstellung A geschwenkt. Dazu muss das Gehäuse zuerst um einen geringen Schwenkwinkel aus der Halteklammer 12 gelöst, sodann um die erste Schwenkachse S1 geschwenkt und zuletzt um die zweite Schwenkachse S2 geschwenkt werden, bis die Bildaufnahmeebene B des Gehäuses etwa planparallel verläuft mit der Empfangsfläche 11 des Bucky. Gleichzeitig wird das Gehäuse um die Linearführung L ausgezogen. Der Arzt arbeitet jetzt ausschliesslich mit dem digitalen Röntgenbildsensor für Echtzeit-Röntgenbilder, während das Bucky 10 inaktiv bleibt.

In den Fig. 9 und 10 ist ein alternatives Röntgengerät dargestellt, bei dem die Strahlenquelle 13 auf an sich bekannte Weise mit Hilfe eines C-Arms 25 direkt dem Bucky zugeordnet ist. Das Bucky 10 kann dabei zusammen mit der Strahlenquelle 13 gedreht werden. Die Verstellbarkeit des Gehäuses 1 zwischen einer Ruhestellung R und einer Arbeitsstellung A ist jedoch gleich wie beim Ausführungsbeispiel gemäss den Figuren 7 und 8.

In einer alternativen Ausführungsform ist die Vorrichtung mit einer Arretierung 17 versehen, mit welcher das Gehäuse 1 in der Arbeitsstellung A und/oder in der Ruhestellung R festgehalten wird. Wie aus Fig. 11 schematisch ersichtlich, steht diese Arretierung 17 mit Steuermitteln 18 in Wirkverbindung. Wenn der Motor M betätigt wird, um das Bucky 10 entlang der Achse S3 zu drehen, wird die Arretierung sofort gelöst. Dann ist das Gehäuse 1um die Achse S1 und/oder S2 schwenkbar.

Wie aus Fig. 12 ersichtlich, ist das Gehäuse 1 vorzugsweise mit einer Schalt- und/oder Anzeigefläche 19 ausgestattet. Über Leuchtdioden auf der Fläche 19 können z.B. Betriebsbereitschaft, Aufnahmezeit und/oder Röntgenstrahlintensität angezeigt werden. Anhand von druckempfindlichen Schaltern auf der Fläche 19 kann z.B. der Röntgenbildsensor manuell ein- oder ausgeschaltet werden oder eine Messdauer eingestellt werden. Zur Vereinfachung der Handhabung ist das Gehäuse vorteilhaft mit einer Griffmulde 20 versehen. Insbesondere das Ein- und Ausfahren des Gehäuses 1 an der Linearführung L wird durch die Griffmulde 20 erleichtert. Auf der Oberfläche des Gehäuses sind in der Bildaufnahmeebene B Positionier-Markierungen 26 aufgetragen.

## Patentansprüche

1. Vorrichtung zum Erzeugen von Echtzeit-Röntgenbildern mit einem sich in einer Bildaufnahmeebene (B) erstreckenden, flächigen Gehäuse (1), in welchem ein digitaler Röntgenbildsensor (2) angeordnet ist, und mit einer Halterung (3) für die Montage des Gehäuses an ein Röntgengerät mit einer Strahlenquelle (13), wobei die Halterung (3) Gelenkmittel (5, 6) aufweist zum Verstellen des Gehäuses (1) zwischen einer Ruhestellung (R) und einer Arbeitsstellung (A), in welcher das Gehäuse (1) im Strahlenfeld der Strahlenquelle (13) angeordnet ist.

2. Vorrichtung nach Anspruch 1, **dadurch gekennzeichnet, dass** die Gelenkmittel ein erstes Gelenk (6) zum Schwenken des Gehäuses um eine erste, vorzugsweise etwa senkrecht zur Bildaufnahmeebene (B) in Ruhestellung (R) des Gehäuses verlaufende Schwenkachse (S1) und ein zweites Gelenk (5) zum Schwenken um eine zweite, etwa senkrecht zur ersten Schwenkachse (S1) verlaufende Schwenkachse (S2) aufweist.

3. Vorrichtung nach Anspruch 2, **dadurch gekennzeichnet, dass** die zweite Schwenkachse (S2) parallel zur Bildaufnahmeebene (B) und /oder einer Seite des Gehäuses (1) verläuft.

4. Vorrichtung nach Anspruch 2 oder 3, **dadurch gekennzeichnet, dass** das Gehäuse (1) über eine Linearführung (L) mit der Halterung (3) verbunden ist und relativ zur zweiten Schwenkachse (S2) zwischen einer ausgezogenen und einer eingeschobenen Position verstellbar ist.

5. Vorrichtung nach einem der Ansprüche 2 bis 4, **dadurch gekennzeichnet, dass** der Schwenkweg um die erste und um die zweite Schwenkachse vorzugsweise wenigstens je 90° beträgt.

6. Vorrichtung nach einem der Ansprüche 1 bis 5, **dadurch gekennzeichnet, dass** das Gehäuse (1) in der Ruhestellung (R) an einer Gehäuseaufnahme (12) feststellbar ist.

7. Vorrichtung nach einem der Ansprüche 1 bis 6, **dadurch gekennzeichnet, dass** die Halterung (3) Mittel zum Fixieren des Gehäuses (1) in der Arbeitsstellung (A) aufweist.

8. Vorrichtung nach einem der Ansprüche 5 oder 6, **dadurch gekennzeichnet, dass** das Gehäuse (1) in der Ruhestellung oder in der Arbeitsstellung mit magnetischen Mitteln, insbesondere mit Permanentmagneten (7), fixierbar ist.

9. Röntgengerät mit einer Strahlenquelle (13) zum Aussenden von Röntgenstrahlen und mit einem Gestell (9), an dem ein Bucky (10) zum Erzeugen digitaler Röntgenbilder derart angeordnet ist, dass eine Empfangsfläche (11) des Bucky auf den Strahlengang der Strahlenquelle (13) ausrichtbar ist,
**dadurch gekennzeichnet, dass** am Gestell (9) eine Vorrichtung zum Erzeugen von Echtzeit-Röntgenbildern nach einem der Ansprüche 1 bis 8 angeordnet ist, wobei die Bildaufnahmeebene (B) des Gehäuses in der Arbeitsstellung etwa planparallel zur Empfangsfläche (11) des Bucky (10) verläuft.

10. Röntgengerät nach Anspruch 9, **dadurch gekennzeichnet, dass**
das Bucky (10) um eine etwa horizontale Achse (S3) drehbar am Gestell (9) gelagert ist und dass die erste Gelenkachse (S1) für das Gehäuse parallel zur Drehachse des Bucky (10) verläuft oder mit dieser zusammenfällt.

11. Röntgengerät nach Anspruch 10, **dadurch gekennzeichnet, dass**
das Bucky (10) und das Gehäuse (1) in der Arbeitsstellung (A) nur gemeinsam schwenkbar bzw. drehbar sind.

12. Röntgengerät nach Anspruch 11, **dadurch gekennzeichnet, dass**
das Bucky (10) über einen Motor (M) drehbar ist, und dass die Vorrichtung zum Erzeugen von Echtzeit-Röntgenbildern arretierbar ist, wobei diese Arretierung mit Steuerungsmitteln (18) in Wirkverbindung steht, welche die Arretierung beim Aktivieren des Motors (M) lösen.
